# EUROPEAN PATENT APPLICATION

(11) **EP 1 231 197 A1**
(43) Date of publication of application: **14.08.2002**
(21) Application number: 02002168.9
(22) Date of filing: 29.01.2002
(51) Int. Cl.: C07C 21/14, C07C 17/02, C07C 315/00, C07C 317/14, C07C 67/00, C07C 69/007

(54) **Process for producing allyl halide compound**

(30) Priority: 07.02.2001 JP 2001030670; 14.02.2001 JP 2001036572; 15.11.2001 JP 2001349769
(71) Applicant: Sumitomo Chemical Company, Limited, Chuo-ku Osaka 541-8550 (JP)
(72) Inventor: Doi, Noriyuki, Ibaraki-shi, Osaka (JP); Seko, Shinzo, Toyonaka-shi, Osaka (JP); Kimura, Kazutaka, Imazato 527,Susono-shi,Shizuoka 410-1104 (JP); Takahashi, Toshiya, Ibaraki-shi, Osaka (JP)
(74) Representative: Henkel, Feiler, Hänzel

(57) **Abstract**

There are disclosed a composition comprising
(E)-1,4-dibromo-2-methyl-2-butene and
(Z)-1,4-dibromo-2-methyl-2-butene, wherein the ratio of the E isomer to the total amount of the E and Z isomers is 0.9 or more; a process for producing the same and a process using the same to produce an allyl halide compound of formula (1): 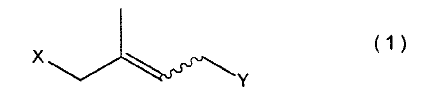 wherein X denotes a bromine atom,
Y denotes an ArS(O)₂ group or an RCOO group,
wherein Ar denotes an aryl group which may be substituted and R denotes a hydrogen atom, a lower alkyl group or an aryl group which may be substituted, and
the wavy line means that the derivative is a mixture of an E or Z geometrical isomer.

## Description

### Field of the Invention

The present invention relates to a process for producing an allyl halide compound (1) as described below, from a dibromo-compound, which is a useful intermediate for the production of pharmaceuticals, feed and food additives such as vitamin A.

### Background of the Invention

There have been disclosed a process of brominating isoprene with bromine in carbon tetrachloride to produce 1,4-dibromo-3-methyl-2-butene (Liebigs Ann. Chem. 283-315 (1988)), and a process of chlorinating isoprene in N,N-dimethylformamide to produce
1,4-dichloro-3-methyl-2-butene (USP 4,001,307). However, the former method was not always satisfactory in that selectivity of the desired
E-isomer in the product was less than 80%, and in the latter method, yield of the desired 1,4-dichloro-3-methyl-2-butene was not satisfactory.

### Summary of the Invention

According to the present invention, the allyl halide compound (1) as described below can be produced in good yield and selectivity of the desired E-isomer.

The present invention provides:
1. a composition comprising (E)-1,4-dibromo-2-methyl-2-butene and (Z)-1,4-dibromo-2-methyl-2-butene, wherein the ratio of the E isomer to the total amount of the E and Z isomers is 0.8 or more;
2. a process for producing the composition as defined above, which comprises reacting isoprene with bromine in the presence of an organic solvent selected from
   an aliphatic or aromatic C2 to C7 hydrocarbon solvent which may be substituted with one or two halogen atoms,
   an aliphatic or aromatic ether solvent,
   an aliphatic or aromatic nitrile solvent, and
   a mixture thereof;
3. a process for producing a composition comprising an allyl halide compound of formula (1): 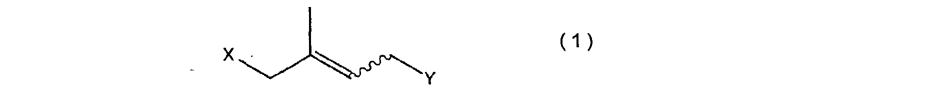 wherein X denotes a bromine atom,
   Y denotes an ArS(O)₂ group or an RCOO group,
      wherein Ar denotes an aryl group which may be substituted and R denotes a hydrogen atom, a lower alkyl group or an aryl group which may be substituted, and
   the wavy line means that the allyl halide compound is a mixture of E-isomer and Z-isomer and the E isomer ratio to the total amount of the E and Z isomers is 0.8 or more,
   which comprises reacting a composition comprising
   (E)-1,4-dibromo-2-methyl-2-butene and
   (Z)-1,4-dibromo-2-methyl-2-butene, wherein the ratio of the E isomer to the total amount of the E and Z isomers is 0.8 or more, with a salt of formula (2)

      YM (2)
   wherein Y represents the same as defined above, and M denotes an alkali metal atom or a quaternary ammonium;
4. a composition comprising an allyl halide compound of formula (1) as defined above; and
5. an allyl halide compound of formula (3) 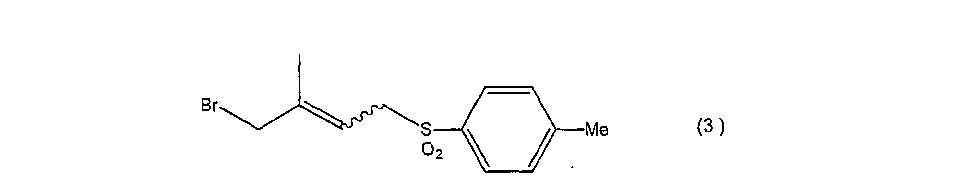
wherein the wavy line means that the allyl halide compound is an E or Z isomer or a mixture thereof.

### Detailed Description of the Invention

The composition comprising (E)-1,4-dibromo-2-methyl-2-butene and (Z)-1,4-dibromo-2-methyl-2-butene as described above can be produced by reacting isoprene with bromine in the presence of the above-described solvent. A preferred composition thereof is a composition comprising (E)-1,4-dibromo-2-methyl-2-butene and (Z)-1,4-dibromo-2-methyl-2-butene, wherein the E-isomer ratio to the total amount of the E and Z isomers is 0.9 or more.

Examples of the aliphatic or aromatic C2 to C7 hydrocarbon solvent which may be substituted with one or two halogen atoms include, for example, 1,2-dichloroethane, n-pentane, n-hexane, cyclohexane, n-heptane, chlorobutane, monochlorobenzene, dichlorobenzene and the like. Among the above-described solvents, preferred are n-pentane, n-hexane, cyclohexane, n-heptane, chlorobutane, monochlorobenzene, dichlorobenzene and the like.

Examples of the aliphatic or aromatic ether solvent include, for example, diethyl ether, 1,4-dioxane, tetrahydrofuran, anisole and the like.

Examples of the aliphatic or aromatic nitrile solvent include, for example, acetonitrile, benzonitrile and the like.

The amount of the organic solvent is not particularly limited, and is preferably 0.5 part or more, more preferably 0.7 part or more by weight per 1 part by weight of isoprene, and the upper limit thereof is preferably 20 parts by weight, more preferably, 10 parts by weight.

The amount of bromine is usually within the range of from 0.001 to 2 moles, preferably from 0.5 to 1 mole per mole of the isoprene.

The reaction of isoprene with bromine may also be conducted in the presence of an inorganic base as an additive. Examples of the inorganic base include, for example,
an alkali metal hydroxide such as lithium hydroxide, sodium hydroxide, potassium hydroxide, or cesium hydroxide,
an alkali metal carbonate such as lithium carbonate, sodium carbonate, potassium carbonate, or cesium carbonate,
an alkali metal hydrogencarbonate such as lithium hydrogencarbonate, sodium hydrogencarbonate, potassium hydrogencarbonate, or cesium hydrogencarbonate.

The amount of the base that may be used is usually within the range of from 0.001 to 1 mole, preferably from 0.01 to 0.2 mole per mole of bromine to be used.

The reaction temperature is usually within the range of from -78°C to 20°C, preferably approximately from -50°C to 20°C, more preferably 10°C or lower, still more preferably 5°C or lower, furthermore preferably 0°C or lower and yet furthermore preferably -10°C or lower. Lower limit of the temperature may be optionally set, within the above-identified range, at such a temperature where the reaction is not adversely affected.

A preferred embodiment of the process include, for example,
a reaction condition where isoprene and bromine are reacted at around 0°C or lower in chlorobutane, thereby the E form of the dihalogene derivative (1) can be obtained in a selectivity of 94% or more.

After completion of the reaction, the dihalogen derivative or a composition thereof can be isolated by a conventional post-treatment, and it may also be further purified by silica gel column chromatography, if necessary.

The dihalogen derivative (composition) thus obtained can be derivatized to an allyl halide compound (1) through the reaction with a salt of formula (2).

In formula (1) and (2), X represents a bromine atom.

Examples of the aryl group represented by Ar or R in formula (1) and (2), includes, for example, a phenyl group or naphthyl group and the like, which may be substituted.

Examples of the substituent on the aryl group, which may be substituted, include, for example, a C1-C5 alkyl group, a C1-C5 alkoxy group, a halogen atom and a nitro group.

Examples of the C1-C5 alkyl group include, for example, methyl, ethyl, n-propyl, isopropyl, n-butyl, isobutyl, sec-butyl, t-butyl, n-pentyl, isoamyl, sec-amyl, t-amyl and the like.

Examples of the C1-C5 alkoxy group include, for example, methoxy, ethoxy, n-propoxy, isopropoxy, n-butoxy, isobutoxy, sec-butoxy, t-butoxy, n-pentyloxy, isoamyloxy, sec-amyloxy, t-amyloxy and the like.

Examples of the halogen atom include, for example, fluorine, chlorine, bromine, and iodine.

Specific examples of the aryl group, which may be substituted with a C1-C5 alkyl group, a C1-C5 alkoxy group, a halogen atom and a nitro group include, for example, phenyl, naphthyl, o-tolyl, m-tolyl, p-tolyl, o-methoxyphenyl, m-methoxyphenyl, p-methoxyphenyl, o-chlorophenyl, m-chlorophenyl, p-chlorophenyl, o-bromophenyl, m-bromophenyl, p-bromophenyl, o-iodophenyl, m-iodophenyl, p-iodophenyl, o-fluorophenyl, m-fluorophenyl, p-fluorophenyl, o-nitrophenyl, m-nitrophenyl, p-nitrophenyl groups and the like.

In formula (1) and (2), examples of the lower alkyl group represented by R in RCOO group with respect to group Y include, for example, a straight or branched C1-C6 alkyl group such as methyl, ethyl, n-propyl, isopropyl, n-butyl, isobutyl, sec-butyl, t-butyl, n-pentyl, isoamyl, sec-amyl, t-amyl and n-hexyl.

The alkali metal or quarternary ammonium (cation) represented by "M" in salt (2) will be described below.

Examples of the alkali metal include, for example, lithium, sodium, potassium and cesium.

Examples of the quaternary ammonium cation include, for example, tetramethylammonium, tetraethylammonium, tetrapropyl ammonium, tetrabutylammonium, tetrapentylammonium, tetrahexylammonium, tetraheptylammonium, tetraoctylammonium, trioctylmethylammonium, tetradecylammonium, tridecylmethylammonium, didecyldimethylammonium, tetradodecylammonium, tridodecylmethylammonium, didodecyldimethylammonium, dodecyltrimethylammonium, dodecyltriethylammonium, tetrahexadecylammonium, hexadecyltrimethylammonium, hexadecyldimethylethylammonium, tetraoctadecylammonium, octadecyltrimethylammonium, octadecyltriethylammonium, benzyltrimethylammonium, benzyltriethylammonium, and benzyltributylammonium.

Specific examples of the salts of carboxylic acids include, for example, lithium acetate, sodium acetate, potassium acetate, cesium acetate, lithium formate, sodium formate, potassium formate, cesium formate, lithium propionate, sodium propionate, potassium propionate, cesium propionate, lithium butyrate, sodium butyrate, potassium butyrate, cesium butyrate, lithium pivalate, sodium pivalate, potassium pivalate, cesium pivalate, lithium benzoate, sodium benzoate, potassium benzoate, cesium benzoate, lithium p-anisate, sodium p-anisate, potassium p-anisate, cesium p-anisate, lithium p-nitorobenzoate, sodium p-nitorobenzoate, potassium p-nitorobenzoate, cesium p-nitorobenzoate, and quaternary ammonium salts of the carboxylic acids as described above. These may contain crystal water. The quarternary ammonium salt of the carboxylic acid can be prepared by neutralization of the corresponding quaternary ammonium hydroxides with the corresponding carboxylic acids.

Examples of the arylsulfinate include, for example, lithium benzenesulfinate, sodium benzenesulfinate, potassium benzenesulfinate, sodium 1-naphthalenesulfinate, sodium 2-naphthalenesulfinate, lithium o-toluenesulfinate, sodium o-toluenesulfinate, potassium o-toluenesulfinate, lithium m-toluenesulfinate, sodium m-toluenesulfinate, potassium m-toluenesulfinate, lithium p-toluenesulfinate, sodium p-toluenesulfinate, potassium p-toluenesulfinate, lithium o-methoxybenzenesulfinate, sodium o-methoxybenzenesulfinate, potassium o-methoxybenzenesulfinate, lithium m-methoxybenzenesulfinate, sodium m-methoxybenzenesulfinate, potassium m-methoxybenzenesulfinate, lithium p-methoxybenzenesulfinate, sodium p-methoxybenzenesulfinate, potassium p-methoxybenzenesulfinate, lithium o-chlorobenzenesulfinate, sodium o-chlorobenzenesulfinate, potassium o-chlorobenzenesulfinate, lithium m-chlorobenzenesulfinate, sodium m-chlorobenzenesulfinate, potassium m-chlorobenzenesulfinate, lithium p-chlorobenzenesulfinate, sodium p-chlorobenzenesulfinate, potassium p-chlorobenzenesulfinate, sodium o-bromobenzenesulfinate, sodium m-bromobenzenesulfinate, sodium p-bromobenzenesulfinate, sodium o-iodobenzenesulfinate, sodium m-iodobenzenesulfinate, sodium p-iodobenzenesulfinate, sodium o-fluorobenzenesulfinate, sodium m-fluorobenzenesulfinate, sodium p-fluorobenzenesulfinate, sodium o-nitrobenzenesulfinate, sodium m-nitrobenzenesulfinate, sodium p-nitrobenzenesulfinate, potassium o-nitrobenzenesulfinate, potassium m-nitrobenzenesulfinate, potassium p-nitrobenzenesulfinate. These may contain crystal water.

The amount of the salt (2) to be used is usually within the range of approximately from 0.2 to 5 moles, preferably from 0.8 to 2 moles, more preferably from 0.8 to 1.2 moles per mole of the total amount of dihalogen derivative.

Examples of the solvent that may be used in this process include, for example,
an aprotic polar solvent such as acetonitrile N,N-dimethylformamide, dimethyl sulfoxide, hexamethylphosphoric triamide, sulfolane, 1,3-dimethyl-2-imidazolidinone, 1-methyl-2-pyrrolidinone, or the like,
an ether solvent such as diethyl ether, tetrahydrofuran, 1,4-dioxane, dimethoxyethane, anisole, diglyme, triglyme, tetraglyme, or the like,
a hydrocarbon solvent such as n-hexane, cyclohexane, n-pentane, benzene, toluene, xylene, or the like, water and
a mixture thereof.

The carboxylic acid salt of formula (2) is preferably reacted in the presence of a phase transfer catalyst.

Examples of the phase transfer catalyst include, for example, quaternary ammonium salts, quaternary phosphonium salt and sulfonium salts.

Examples of the quaternary ammonium salts include, for example, tetramethylammonium chloride, tetraethylammonium chloride, tetrapropylammonium chloride, tetrabutyl ammonium chloride, tetrapentylammonium chloride, tetrahexylammonium chloride, tetraheptylammonium chloride, tetraoctylammonium chloride, trioctylmethylammonium chloride, tetradecylammonium chloride, tridecylmethylammonium chloride, didecyldimethylammonium chloride, tetradecylammonium chloride, tridecylmethylammonium chloride, didocecyldimethylammonium chloride, dodecyltrimethylammonium chloride, dodecyltriethylammonium chloride, tetrahexadecylammonium chloride, hexadecyltrimethylammonium chloride, hexadecyldimethylethylammonium chloride, tetraoctadecylammonium chloride, octadecyltrimethylammonium chloride, octadecyltriethylammonium chloride, benzyltrimethyammonium chloride, benzyltriethylammonium chloride, benzyltributylammonium chloride, 1-methylpyridinium chloride, 1-hexadecylpyridinium chloride, 1,4-dimethylpyridinium chloride, trimethylcyclopropylammonium chloride, or compounds resulting from changing the chlorides to the corresponding bromides, iodides and hydrogensulfates.

Examples of the quaternary phosphonium salt include, for example, tributylmethylphosphonium chloride, triethylmethylphosphonium chloride, methyltriphenoxyphosphonium chloride, butyltriphenylphosphonium chloride, tetrabutylphosphonium chloride, benzyltriphenylphosphonium chloride, tetraoctylphosphonium chloride, hexadecyltrimethylphosphonium chloride, hexadecyltributylphosphonium chloride, hexadecyldimethylethylphosphonium chloride, tetraphenylphosphonium chloride, or compounds resulting from changing the chlorides to the corresponding bromides and iodides.

Examples of the sulfonium salts include, for example, benzylmethylethylsulfonium chloride, benzyldimethylsulfonium chloride, benzyldiethylsulfonium chloride, dibutylmethylsulfonium chloride, trimethylsulfonium chloride, triethylsulfonium chloride, tributylsulfonium chloride, or sulfonium bromides and iodides corresponding to the above-described chloride.

The phase transfer catalyst is usually used in an amount of approximately from 0.001 to 0.3 mole, preferably approximately from 0.05 to 0.2 mole per mole of the (E/Z)-1,4-dibromo-2-methyl-2-butene.

Alternatively, the reaction can be carried out in a two-phase system comprising a hydrophobic organic solvent (e.g, hydrocarbon solvents as described above) and water.

The reaction temperature is usually within the range of from -30°C to the boiling point of the solvent used, preferably approximately from -10°C to 60°C.

After completion of the reaction, the allyl halide compound (1) can be isolated by a conventional post-treatment such as extraction with a water-immiscible solvent, phase separation, and evaporation, and the isolated product may be further purified by silica gel column chromatography or the like, if necessary.

In the reaction described above, the geometric configuration with respect to the double bond of the dihalogen derivative is retained.

The allyl halide compound (1) and (3) thus obtained can be readily derivatized to a vitamin A derivative, for example, in a similar manner by a synthetic route disclosed in Helv. Chim. Acta 59, 387 (1976) and a synthetic route shown in the following scheme 1:

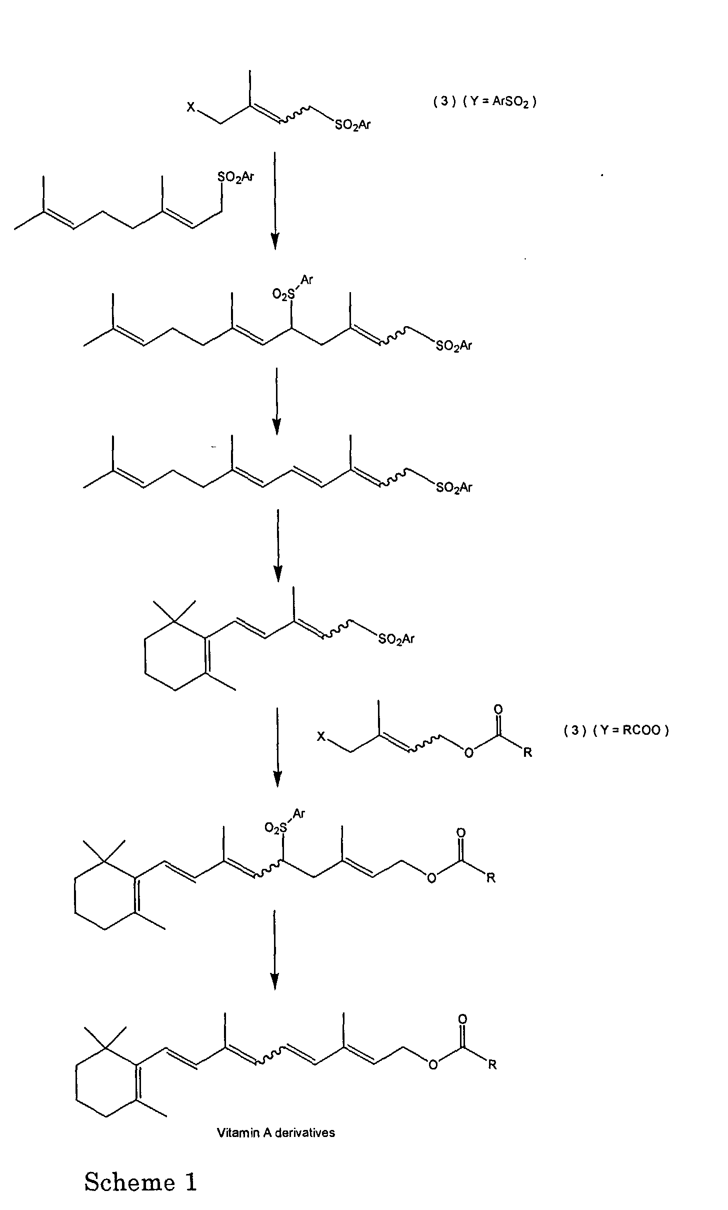

### EXAMPLES

The present invention will be described in more detail by reference to Examples below, but the invention is not limited to the Examples.

### Example 1

To a solution of 37.4 g (549 mmol) of isoprene in 500 ml of chlorobutane was dropwise added 43.9 g (275 mmol) of bromine at a temperature range of from -50°C to -30°C, and the resulting reaction solution was maintained for 2.5 hours at the temperature. After the reaction solution was poured into water, an organic layer was separated and washed sequentially with a diluted aqueous sodium thiosulfate solution, a diluted aqueous sodium hydrogencarbonate solution and saturated brine. The organic layer was dried over anhydrous sodium sulfate, and filtered solution was evaporated to give a crude 1,4-dibromo-2-methyl-2-butene. Analysis of the obtained crude product by gas chromatography showed that 1,4-dibromo-2-methyl-2-butene was obtained in a yield of 86% (E/Z = 96/4).

### Example 2

To 30 ml of n-hexane, which was dehydrated with molecular sieves 3A, were added 4.11 g (60 mmol) of isoprene and 0.84 g (6 mmol) of potassium carbonate and the resulting mixture was cooled to 0°C. To the mixture was dropwise added 4.82 g (30 mmol) of bromine through a dropping funnel and the resulting mixture was stirred for 2.5 hours at the temperature. The reaction solution was added to water, and a separated organic layer was washed with saturated brine. After the organic layer was dried over anhydrous sodium sulfate, the organic layer was filtered and the filtrate was evaporated to give 1,4-dibromo-2-methyl-2-butene. Analysis of the obtained crude product by gas chromatography showed that 1,4-dibromo-2-methyl-2-butene was obtained in a yield of 73% (E/Z = 94/6).

### Example 3

To a solution of 0.78 g (purity 93.1%, 3.2 mmol, E/Z = 94/6) of 1,4-dibromo-2-methyl-2-butene in 4 ml of N,N-dimethylformamide, cooled to 0°C, were added 1 ml of water, 0.34 g (3.3 mmol) of lithium acetate dihydrate and 2 ml of N,N-dimethylformamide in this order and stirred at the temperature for 5 hours. After completion of the reaction, water was added thereto and extracted with ethyl acetate. A separated organic layer was washed with water and saturated brine sequentially. After drying over anhydrous sodium sulfate, the organic layer was filtered and the filtrate was evaporated to give 1-acetoxy-4-bromo-3-methyl-2-butene. Analysis of the obtained crude product by gas chromatography showed that 1-acetoxy-4-bromo-3-methyl-2-butene was obtained in a yield of 67% (E/Z = 96/4).

### Example 4

1-Acetoxy-4-bromo-3-methyl-2-butene was obtained in a yield of 48% (E/Z = 95/5) by conducting a reaction in a similar manner as in Example 3 except that 6.0 mmol of sodium acetate was used in place of 6.0 mmol of lithium acetate dihydrate.

### Example 5

To a solution of 0.70 g (purity 98.0%, 3.0 mmol, E/Z = 97/3) of 1,4-dibromo-2-methyl-2-butene in 6 ml of acetonitrile was added 0.32 g (3.3 mmol) of potassium acetate, and then the mixture was heated to 40°C and was stirred at the temperature for 7 hours. After completion of the reaction, water was added thereto and extracted with ethyl acetate. A separated organic layer was washed with water and saturated brine. After being dried over anhydrous sodium sulfate, the organic layer was filtered and the filtrate was evaporated to give crude 1-acetoxy-4-bromo-3-methyl-2-butene. Analysis of the obtained crude product by gas chromatography showed that was obtained in a yield of 62% (E/Z = 98/2).

### Example 6

To 2 ml of acetonitrile were added 236 mg (purity 94.3 %, 1.0 mmol, E/Z = 92/8) of 1,4-dibromo-2-methyl-2-butene, 90 mg (1.1 mmol) of sodium acetate and 32 mg (0.1 mmol) of tetrabutylammonium bromide and stirred at 50°C for 6 hours. After completion of the reaction, saturated aqueous sodium hydrogencarbonate solution was added thereto and extracted with ethyl acetate. A separated organic layer was washed with saturated brine. After being dried over anhydrous sodium sulfate, the organic layer was filtered and the filtrate was evaporated to give crude 1-acetoxy-4-bromo-3-methyl-2-butene. Analysis of the obtained crude product by gas chromatography showed that 1-acetoxy-4-bromo-3-methyl-2-butene was obtained in a yield of 58% (E/Z = 91/9).

### Example 7

To a mixture of 2 ml of toluene and 0.5 ml of water were added 236 mg (purity 94.3 %, 1.0 mmol, E/Z = 92/8) of 1,4-dibromo-2-methyl-2-butene, 90 mg (1.1 mmol) of sodium acetate and 34 mg (0.1 mmol) of tetrabutylphosphonium bromide, and the resulting reaction mixture was stirred at 50°C for 8.5 hours. After completion of the reaction, a saturated aqueous sodium hydrogencarbonate solution was added thereto and extracted with ethyl acetate. A separated organic layer was washed with saturated brine. After being dried over anhydrous sodium sulfate, the organic layer was filtered and the filtrate was evaporated to give 1-acetoxy-4-bromo-3-methyl-2-butene. Analysis of the obtained crude product by gas chromatography showed that 1-acetoxy-4-bromo-3-methyl-2-butene was obtained in a yield of 54% (E/Z = 90/10).

### Example 8

To a mixed solvent of 2 ml of toluene and 0.5 ml of water were added 236 mg (purity 94.3 %, 1.0 mmol, E/Z = 82/18) of 1,4-dibromo-2-methyl-2-butene, 378 mg (purity 60%, 1.2 mmol) of tetraethylammonium acetate and 34 mg (0.1 mmol) of tetrabutylphosphonium bromide, and the resulting mixture was stirred at 50°C for 9 hours. After completion of the reaction, saturated brine was added thereto and extracted with ethyl acetate. A separated organic layer was dried over anhydrous sodium sulfate and filtered. The filtrate was evaporated to give 1-acetoxy-4-bromo-3-methyl-2-butene. Analysis of the obtained crude product by gas chromatography showed that 1-acetoxy-4-bromo-3-methyl-2-butene was obtained in a yield of 44% (E/Z = 88/12).

### Example 9

To a solution of 0.71 g (purity 96.7%, 3.0 mmol, E/Z = 93/7) of 1,4-dibromo-2-methyl-2-butene in 6 ml of N,N-dimethylformamide was added 0.48 g (3.3 mmol) of sodium benzoate, and then the resulting mixture was stirred at 40°C for 3.5 hours. After completion of the reaction, water was added thereto and extracted with ethyl acetate. A separated organic layer was washed with water and saturated brine. After being dried over anhydrous sodium sulfate, the organic layer was filtered and the filtrate was evaporated to give 4-bromo-3-methyl-2-butenyl benzoate. The analysis of the obtained crude product by gas chromatography showed that 4-bromo-3-methyl-2-butenyl benzoate was obtained in a yield of 61% (E/Z = 97/3).

### Example 10

To a solution of 0.71 g (purity 96.7%, 3.0 mmol, E/Z = 93/7) of 1,4-dibromo-2-methyl-2-butene in 6 ml of N,N-dimethylformamide was added 0.48 g (3.3 mmol) of sodium butyrate, and then the mixture was stirred at 30°C for 3.5 hours. After completion of the reaction, water was added thereto and extracted with ethyl acetate. A separated organic layer was washed with water and saturated brine. After being dried over anhydrous sodium sulfate, the organic layer was filtered and the filtrate was evaporated to give 4-bromo-3-methyl-2-butenyl butyrate. Analysis of the obtained crude product by gas chromatography showed that 4-bromo-3-methyl-2-butenyl butyrate was obtained in a yield of 54% (E/Z = 95/5).

### Example 11

To a solution of 6.24 g (purity 93.1%, 25.5 mmol) of 1,4-dibromo-2-methyl-2-butene in 30 ml of acetonitrile was added a solution of 4.90 g (27.5 mmol) of sodium p-toluenesulfinate in 30 ml of water. Subsequently, the resulting mixture was heated to 50°C and was stirred for 2 hours. After completion of the reaction, water was added thereto and extracted with ethyl acetate. A separated organic layer was washed with saturated brine. After being dried over anhydrous sodium sulfate, the organic layer was filtered and the filtrate was evaporated to give 1-(p-toluenesulfonyl)-3-methyl-4-bromo-2-butene. Analysis of the obtained crude product by gas chromatography showed that
1-(p-toluenesulfonyl)-3-methyl-4-bromo-2-butene was obtained in a yield of 59%(E/Z=99/1).
1-(p-Toluenesulfonyl)-3-methyl-4-bromo2-butene
¹H-NMR δ 1.47(s, 3H), 2.45(s, 3H), 3.81 (d, J=8.0Hz, 2H), 3.90(s, 3H), 5.62(t, J=8.0Hz, 1H), 7.35(d, J=8.3Hz, 2H), 7.75(d, J=8.3Hz, 2H). ¹³C-NMR δ 15.22, 22.06, 39.23, 56.47, 116.87, 128.86, 130.27, 135.84, 141.90, 145.27.

### Example 12

To a solution of 200 mg (1 mmol) of sodium benzenesulfinate dihydrate dissolved in 2 ml of water was dropwise added a solution of 261 mg (purity 87.3%, 1 mmol) of 1,4-dibromo-2-methyl-2-butene in 2 ml of acetonitrile. The resulting mixture was stirred at room temperature for 10 hours, followed by addition of water and then extraction with ethyl acetate. A separated organic layer was washed with saturated brine. After being dried over anhydrous sodium sulfate, the organic layer was filtered and the filtrate was evaporated to give
1-(phenylsulfonyl)-3-methyl-4-bromo-2-butene. Analysis of the obtained crude product was purified by silica gel column chromatography to give 1-(phenylsulfonyl)-3-methyl-4-bromo-2-butene in a yield of 58% (E/Z=99/1).

## Claims

1. A composition comprising (E)-1,4-dibromo-2-methyl-2-butene and (Z)-1,4-dibromo-2-methyl-2-butene, wherein the ratio of the E isomer to the total amount of the E and Z isomers is 0.8 or more.

2. A composition according to claim 1, wherein the E isomer ratio to the total amount of the E and Z isomers of 1,4-dibromo-2-methyl-2-butene is 0.9 or more.

3. A process for producing a composition comprising (E)-1,4-dibromo-2-methyl-2-butene and (Z)-1,4-dibromo-2-methyl-2-butene,
which comprises reacting isoprene with bromine in the presence of an organic solvent selected from
an aliphatic or aromatic C2 to C7 hydrocarbon solvent which may be substituted with one or two halogen atoms,
an aliphatic or aromatic ether solvent, and
an aliphatic or aromatic nitrile solvent,
wherein the ratio of the (E)-1,4-dibromo-2-methyl-2-butene to the total amount of the E and corresponding Z isomers is 0.8 or more.

4. A process according to claim 3, wherein isoprene is reacted with bromine at a temperature range of -10°C or lower.

5. A process according to claim 4, wherein the reaction is conducted at a temperature range of from -20°C or lower.

6. A process according to claim 3, 4, or 5, wherein bromine is reacted with isoprene in the presence of an inorganic base.

7. A process according to claim 6, wherein the amount of the inorganic base is from 0.01 to 0.2 mole per mol of bromine.

8. A process according to claim 3, wherein the organic solvent is a solvent selected from n-pentane, n-hexane, cyclohexane, n-heptane, chlorobutane, monochlrorobenzene, and dichlorobenzene.

9. A process according to claim 3, which further comprises reacting the dihalogen derivative, with
a salt of formula (2)
YM (2)
wherein Y represents the same as defined above, and M denotes an alkali metal atom or a quaternary ammonium, to produce an allyl halide compound of formula (1): 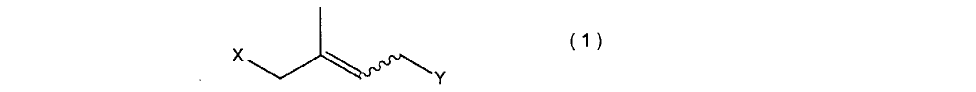 wherein X denotes a bromine atom,
Y denotes an ArS(O)₂ group or an RCOO group,
wherein Ar denotes an aryl group which may be substituted and R denotes a hydrogen atom, a lower alkyl group or an aryl group which may be substituted, and the E isomer ratio to the total amount of the E and Z isomers of 1,4-dibromo-2-methyl-2-butene is 0.8 or more.

10. A process according to claim 9, wherein Ar and R independently represent a phenyl or naphthyl group which may be substituted with a member selected from a C1-C5 alkyl group, a C1-C5 alkoxy group, a halogen atom, a nitro group, or R represents a hydrogen atom, a C1-C6 straight or branched alkyl group.

11. A process according to claim 9 or 10, wherein Y represents the RCOO group.

12. A process according to claim 9, wherein the 1,4-dibromo-2-methyl-2-butene is reacted with the salt of formula (2) in the presence of a phase transfer catalyst.

13. A process according to claim 12, wherein the phase transfer catalyst is a quaternary ammonium salt, a quaternary phosphonium salt or a sulfonium salt.

14. A process according to claim 9, wherein Y denotes the ArS(O)₂ group.

15. A process for producing a composition comprising an allyl halide compound of formula (1): 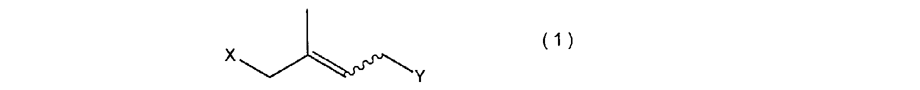 wherein
X denotes a bromine atom,
Y denotes an ArS(O)₂ group or an RCOO group,
wherein Ar denotes an aryl group which may be substituted and R denotes a hydrogen atom, a lower alkyl group or an aryl group which may be substituted, and the wavy line means that the compound is a mixture of E and Z isomers and the E isomer ratio to the total amount of the E and Z isomers of formula (3) is 0.8 or more,
which comprises reacting a composition comprising
(E)-1,4-dibromo-2-methyl-2-butene and
(Z)-1,4-dibromo-2-methyl-2-butene, wherein the ratio of the E isomer to the total amount of the E and Z isomers is 0.8 or more, with a salt of formula (2)
YM (2)
wherein Y represents the same as defined above, and M denotes an alkali metal atom or a quaternary ammonium.

16. A composition comprising an allyl halide compound of formula (1): 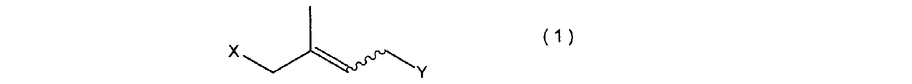 wherein
X denotes a bromine atom,
Y denotes an ArS(O)₂ group or an RCOO group,
wherein Ar denotes an aryl group which may be substituted and R denotes a hydrogen atom, a lower alkyl group or an aryl group which may be substituted, and
the wavy line means that the compound is a mixture of E and Z isomers, and the E isomer ratio to the total amount of the E and Z isomers of formula (1) is 0.8 or more.

17. A composition according to claim 16, wherein the E isomer ratio to the total amount of the E and Z isomers of formula (1) is 0.9 or more.

18. An allyl halide compound of formula (3): 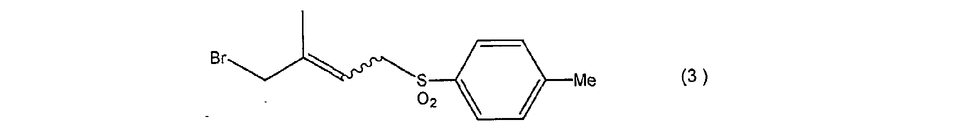 wherein the wavy line means that the allyl halide compound is an E or Z isomer or a mixture thereof.
